# EUROPEAN PATENT APPLICATION

(11) **EP 1 634 550 A2**
(43) Date of publication of application: **15.03.2006**
(21) Application number: 05108346.7
(22) Date of filing: 12.09.2005
(51) Int. Cl.: A61F 2/46, A61B 17/16, A61B 19/00

(54) **Checking jig**

(30) Priority: 13.09.2004 GB 0420347
(71) Applicant: Finsbury (Development) Limited, Leatherhead, Surrey KT22 0BA (GB)
(72) Inventor: Wozencroft, Robert, Michael, Epsom, Surrey KT19 8SS (GB); Tuke, Michael Anthony, Guilford, Surrey GU1 3TF (GB)
(74) Representative: Smaggasgale, Gillian Helen

(57) **Abstract**

A checking jig comprising: a body (2) having an aperture therethrough which in use can be placed over a guide wire located in a well in the head of the femur; an arm (3) extending from the body and shaped to extend around the head of the femur; a tip (4) located on the end of the arm remote from the body, said tip representing the diameter at which a particular size of sleeve cutter will cut; and means for adjusting the position of the tip between different positions representing different cut diameters.

## Description

The present invention relates to a tool for use in hip resurfacing operations. More particularly, it relates to a checking jig which may be used to verify the correct positioning and size of sleeve cutter to be used in machining the head of a femur.

The efficient functioning of the hip joints is extremely important to the well being and mobility of the human body. Each hip joint is comprised by the upper portion of the upper leg bone (femur) which terminates in an offset bony neck surmounted by a ball-headed portion which rotates within a socket, known as the acetabulum, in the pelvis. Diseases such as rheumatoid- and osteo-arthritis can cause erosion of the cartilage lining of the acetabulum so that the ball of the femur and the hip bone rub together causing pain and further erosion Bone erosion may cause the bones themselves to attempt to compensate for the erosion which may result in the bone being reshaped. This misshapen joint may cause pain and may eventually cease to function altogether.

Operations to replace the hip joint with an artificial implant are well-known and widely practiced. Generally, the hip prosthesis will be formed of two components, namely: an acetabular, or socket, component which lines the acetabulum; and a femoral, or stem, component which replaces the femoral head. During the surgical procedure for implanting the hip prosthesis the cartilage is removed from the acetabulum using a reamer such that it will fit the outer surface of the acetabular component of the hip prosthesis. The acetabular component can then be inserted into place. In some arrangements, the acetabular component may simply be held in place by a tight fit with the bone. However, in other arrangements, additional fixing means such as screws or bone cement may be used. The use of additional fixing means help to provide stability in the early stages after the prosthesis has been inserted. In some modern prosthesis, the acetabular component may be coated on its external surface with a bone growth promoting substance which will assist the bone to grow and thereby assist the holding of the acetabular component in place. The bone femoral head will be removed and the femur hollowed using reamers and rasps to accept the prosthesis. The stem portion will then be inserted into the femur.

In some cases, a femoral component of the kind described above may be replaced with components for use in femoral head resurfacing or for use in thrust plate technology.

Although the prosthesis being inserted when the head is being replaced or resurfaced or in thrust plate arrangements is relatively small, the requirement for the surgeon to obtain the necessary access to the hip joint means that it is necessary to make a large incision on one side of the hip. In one technique, a straight incision is made through the skin on the posterior edge of the greater trochanter. In some techniques this incision may be made when the hip is flexed to 45°. By known techniques, the muscles and tendons are parted and held by various retractors such that they do not interfere with the surgeons access to the hip joint. The hip is then dislocated to provide access to the head of the femur.

It will be acknowledged that it is essential that the replacement surface for the head of the femur should be precisely located in both angular and translation positions of the axis of the femoral neck of the implant. To assist this, in some techniques, the surgeon inserts a pin in the lateral femur. The desired position of the pin will be known from pre-operative analysis of the x-rays. The surgeon will measure the desired distance down the femur from the tip of the greater trochanter and the alignment pin is inserted through the vastus lateralis fibres. The alignment pin is inserted in a transverse direction into the mid-lateral cortex and directed upwardly towards the femoral head. The pin is left protruding so that an alignment guide can be hooked over the alignment pin. Suitable alignment guides include those known as the McMinn Alignment Guide available from Midland Medical Technologies Ltd.

These alignment guides of the kind described above generally comprise a hook or aperture which is placed over the alignment pin thus providing a good angular position for the axis of the implant in valgus, varus and ante-version of the neck. The guide will then be adjusted such that a cannulated rod is located such that the aperture therein is directed down the mid-lateral axis of the femoral neck. A stylus having been set to the desired femoral component size is positioned such that it can be passed around the femoral neck.

When the stylus can be passed around the femoral neck, the cannulated rod is locked in position. Once the guide is stabilised in this way fine adjustments can be made until the surgeon is happy that the guide is in the required position.

A guide wire can then be inserted though the cannulated rod. This guide wire is then used in the further surgery in which the femoral head is shaped to accept the prosthesis. This shaping involves removing the top of the head at an appropriate position and then machining the sides of the head using a sleeve cutter. These sleeve cutters are arranged such that the diameter cut will be correct for the replacement head size chosen and will bottom on the top of the cut head such that the teeth of the cutter do not dangerously over-sail the head-neck junction and cause soft tissue damage or neck notching.

Thus the machining procedure usually comprises the steps of drilling a well into the head of the femur, removing the drill, removing the top of the head of the femur, inserting a guide rod into the well, locating a sleeve cutter on the guide rod and cutting the head and optionally chamfer cutting the head. However, it will be understood, that the order of the steps may be altered.

An alignment guide is generally used to ensure that the aperture drilled in the femoral head is both central to the femoral neck and at the correct angle of alignment to the femoral neck and that the shaping of the femoral head is accurate for the chosen head size.

It will therefore be understood that it is very important that the alignment guide is positioned correctly. Failure to do so may have the disastrous effect of allowing the machining of the cylinder of the head during the shaping procedure to "notch" into the neck of the femur. This will predispose the bone to early failure on load bearing.

Alternative improved alignment guides are described in co-pending UK applications 0408792 and 0408793 filed 20th April 2004, 0419494 filed 2nd September 2004 and 0419640 filed 3rd September 2004. These improved alignment guides allow the required incision in the hip to be as small as possible and the amount of interaction with healthy tissue to be minimised. This is achievable as they do not require the alignment pin required by previous devices to be inserted. Where these guides are used, all of the surgical procedure takes place at the femoral head.

Other guides are known which are, in use, located on the femoral neck itself. These are used in a similar manner to those described above and may involve some adjustment by the surgeon before he selects the best position.

Whichever alignment guide is used, it is desirable that the surgeon can satisfy himself that the size of sleeve cutter he has selected to machine the head of the femur, whilst machining the head to the required size will not impinge on the neck of the femur with the risk that this could be notched. Checking tools have been proposed for this purpose however these can be cumbersome to use and do not generally allow the surgeon to readily check a variety of cutting sizes.

There is therefore a requirement for an improved checking tool which readily enables a range of cutting sizes to be verified.

According to the present invention there is provided a checking jig comprising: a body having an aperture therethrough which in use can be placed over a guide wire located in a well in the head of the femur; an arm extending from the body and shaped to extend around the head of the femur; a tip located on the end of the arm remote from the body said tip representing the diameter at which a particular size of sleeve cutter will cut; and means for adjusting the position of the tip between different positions representing different cut diameters.

In a preferred arrangement the different positions will be a selection of preset positions such that there is not a sliding scale but definitive positions. Commercially available heads are sold in a range of sizes to suit the needs of individual patients. Generally head sizes are from 38 mm to 58 mm in diameter, with 2 mm increments between neighboring sizes. However, various ranges of head sizes may be covered.

The arrangement of the present invention preferably allows the tip to be positioned to represent the internal diameter of sleeve cutters which correspond to these head sizes. Thus each of the preset positions corresponds to one of the varying diameters. In a preferred arrangement the full range of diameters is encompassed by the range of preset positions.

Once the jig of the present invention is in position, the surgeon can rotate the jig using the guide wire as an axis such that the tip will circumscribe the cutting diameter. This enables the surgeon to visualise the size of cut and immediately note if the cut would impinge on the neck.

The means for adjusting the position of the tip may be located on or within the body or it may be located on or within the arm.

In the embodiment in which the means for adjusting the position of the tip is located on or in the body, the body itself may be formed from a fixed first member and a movable second member which is connectable to the first member and movable with respect thereto. The arm may be connected to one of the first and second member and the other of the first and second member will include an aperture through which, in use, the guide wire can be placed such that the relative position of the aperture in one of the first and second members to the tip of the arm attached to the other of the first and second member will represent the internal radius of the sleeve cutter and will be adjustable.

In one arrangement one of the first and second member is a collar in which the second member is a sliding fit.

The relative movement may be lateral along a substantially straight line or it may be rotational, for example around an axis which passes through the centre of the body.

It will be understood that in the embodiment where the relative movement is rotational, as the aperture is moved around the axis, its distance from the tip is altered.

In one arrangement the jig of the present invention comprises a body which comprises a collar means having an arm extending therefrom which is shaped to extend round the head of a femur. The arm has a tip located at the end of the arm. The body also includes a second member which is a sliding fit in the collar. The second member may be a rotatable jog dial which has a skirt that is a sliding fit in the collar. Means may be provided to prevent the second member from being removed from the collar when the jig is in use.

Any suitable means may be provided to enable the jog dial to move between the preset locations. One suitable means is a spring ball grub screw mechanism which will interact with indexing slots in the jog dial.

The body may include an elongated cannulated rod extending upwardly from the body such that the bore of the cannulated rod cooperates with the aperture in the body to improve the stability of the jig on the guide wire. For ease of storage, the cannulated bore may be telescopic.

The aperture and any bore in the cannulated rod may be sized not only to connect over a guide wire but also to enable the jig to be placed over the guide rod which will be inserted into the enlarged bore in the femoral head during the machining process. This will enable the jig of the present invention to be used as a further check once the femoral head has been inserted which may be after the head has been resected. In an additional or alternative arrangement, the central portion of the slidable telescopic cannulated rod enables the size of the bore of the cannulated rod to be adjusted. Thus when the central rod is in position, the bore will be an appropriate size to surround the guidewire and when removed, the bore is increased so that the bore will accommodate the guide rod.

In an alternative arrangement, the body may comprise a cannulated rod which in use will be placed over the guidewire. The portion of the cannulated rod which will be adjacent to the head may include teeth to assist the cannulated rod to engage the femoral head. The arm may be connected to the cannulated rod by means of a sleeve around the rod or it may fit into bore of the rod. In this arrangement, the adjusting means will generally be located on the arm. The arm may be adjustable not only as to the radius that the tip will travel when the jig is rotated but also as to the length of the arm. In this arrangement a cutting guide representing the position at which the head of the femur should be cut may be included such that when the tip is positioned at the head/neck junction, the cutting guide is in the correct position to facilitate accurate cutting.

The present invention will now be described by way of example with reference to the accompanying drawings in which:
- Figure 1: illustrates one embodiment of the jig of the present invention;
- Figure 2: illustrates the components separated;
- Figure 3: illustrates how the jig may be assembled to facilitate its connection to the guide wire and its location around the femoral head;
- Figure 4: represents the jig of the present invention in location on a femoral head;
- Figure 5: illustrates the use of the jig as a further check on a machined femoral head;
- Figure 6: is a schematic illustration of the index position;
- Figure 7: illustrates the movement of the aperture and its relationship to the radius of the jig;
- Figure 8: is a schematic diagram of a jig of the alternative embodiment of the present invention; and
- Figure 9: is a close-up of a portion of the device.

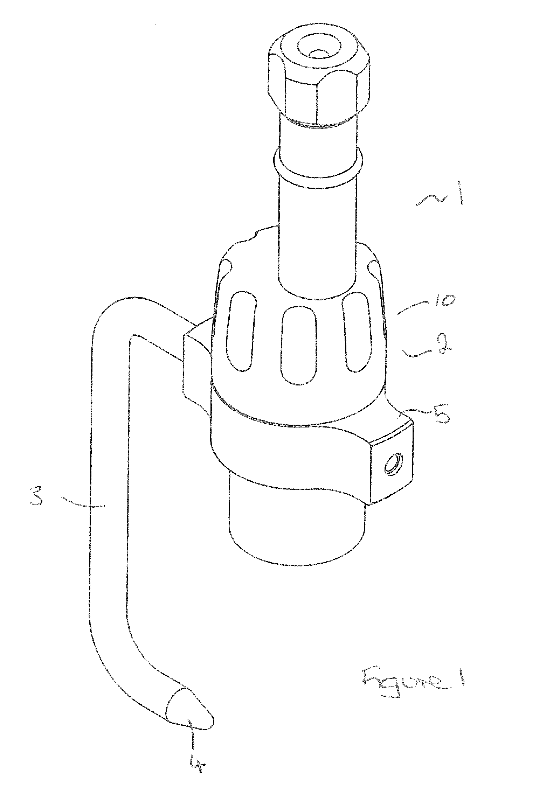

As illustrated in Figure 1, the jig 1 of the present invention includes a body 2 having an arm 3 extending therefrom. A tip 4 is located at the free end of the arm.

In the illustrated arrangement the body comprises a collar 5 in which the rotating component 10 which is a jog dial is placed.

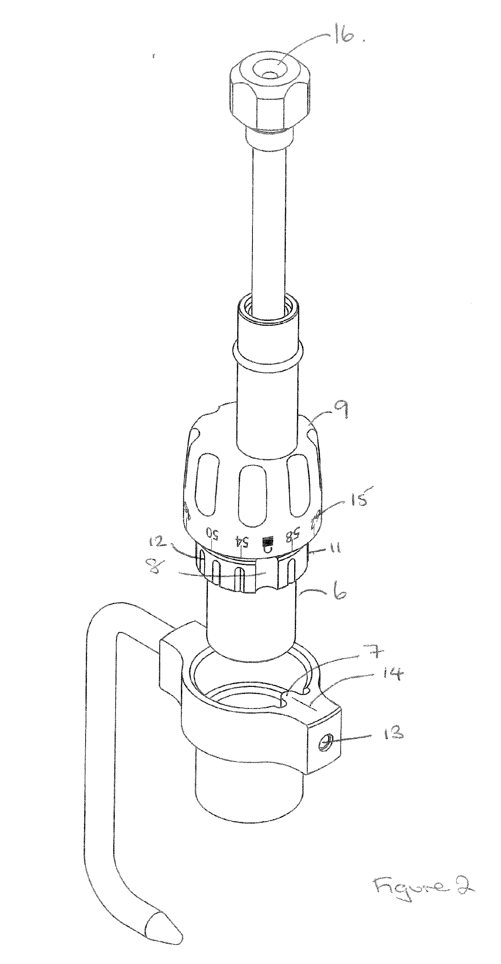

As illustrated in Figure 2 the jog dial comprises a seating member 6. A ring 11 is located around the seating member and includes spaced around its periphery a selection of indexing slots 12. A release slot 8 is provided which will be aligned to a stop 7 in the collar which allows the jog dial to be inserted and removed. When the jog dial is rotated, the release slots 12 interlock in turn with a spring ball grub screw (not shown) which is located in an aperture 13 in the collar. A set radius locator 14 is provided on the collar and the user will select the required position by lining up the selected portion with the locator. Indicia 15 are marked around the jog dial.

An aperture (not shown) in the body is aligned with the bore of the cannulated rod 16 through which the guide wire can be passed. The rod is telescopic from an elongated position as illustrated in Figure 2 to the reduced position of Figure 1. In an additional or alternative arrangement, the central portion of the slidable telescopic cannulated rod enables the size of the bore of the cannulated rod to be adjusted. Thus when the central rod is in position, the bore will be an appropriate size to surround the guidewire and when removed, the bore is increased so that the bore will accommodate the guide rod.

The ability to separate the components assist the location of the jig on the guidewire. Due to the shaping of the arm, the jig while completely assembled cannot simply be placed onto the guidewire. The large size of the aperture 17 in the collar 5, the arm can be maneuvered around the head. Once in the correct orientation, the remainder of the jog can be slid along the guidewire and seated into the collar. It will be understood that alternative arrangements may be used to enable the jig to be located.

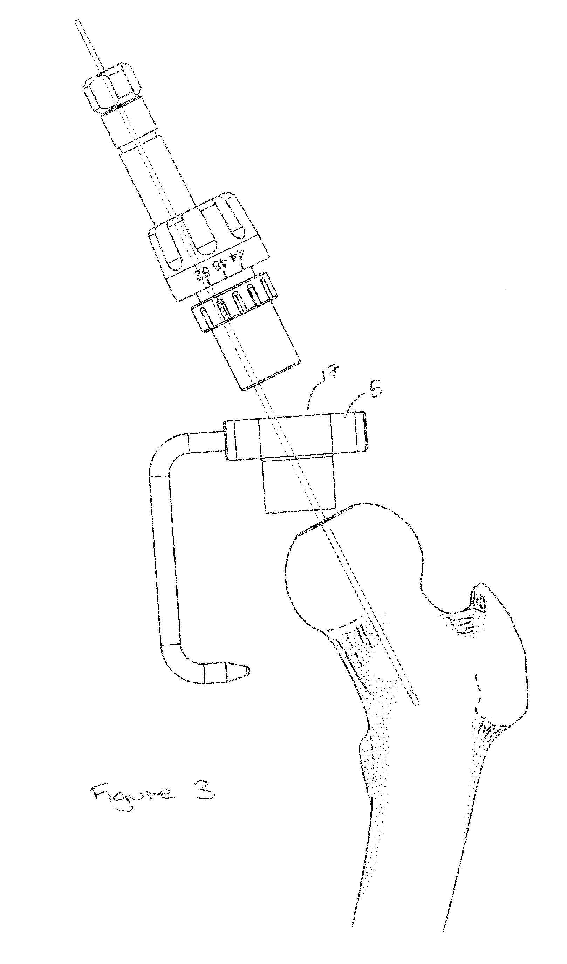

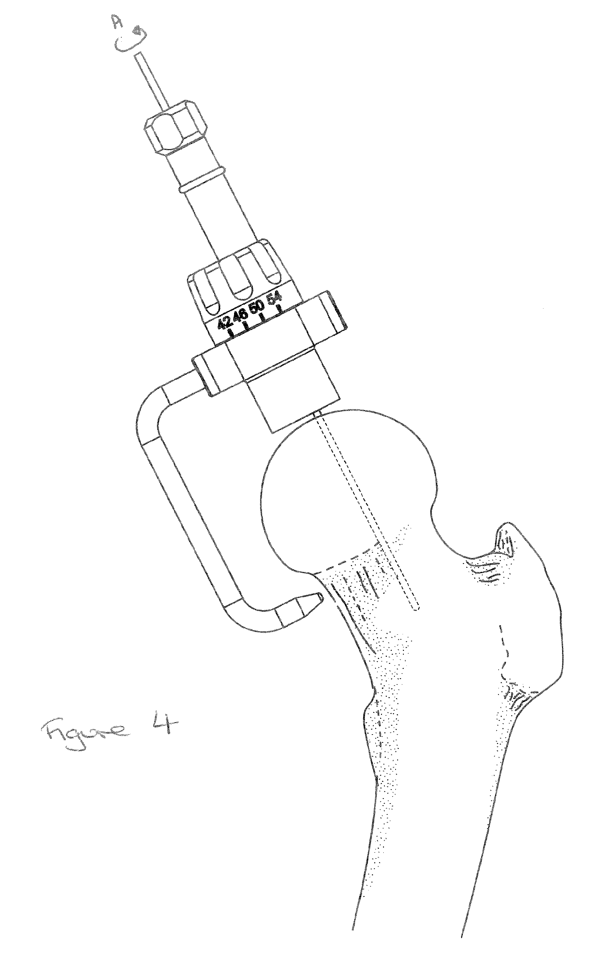

The jig located in position is illustrated in Figure 4. The required position of the aperture can then be selected by rotating the jog dial. Once the required size is selected, the jig may be rotated around the guidewire as illustrated at A and the surgeon can view whether the tip interacts with the neck at any position.

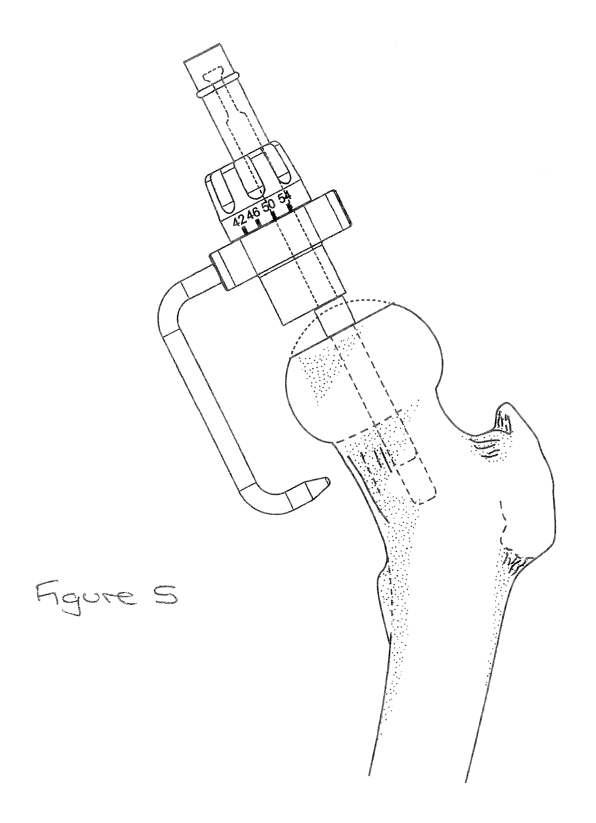

Once the head has been resected and a guide rod is located into the head of the femur, the internal portion of the cannulated rod may be removed so that the jig can be placed over the rod and used for further checks as illustrated in Figure 5.

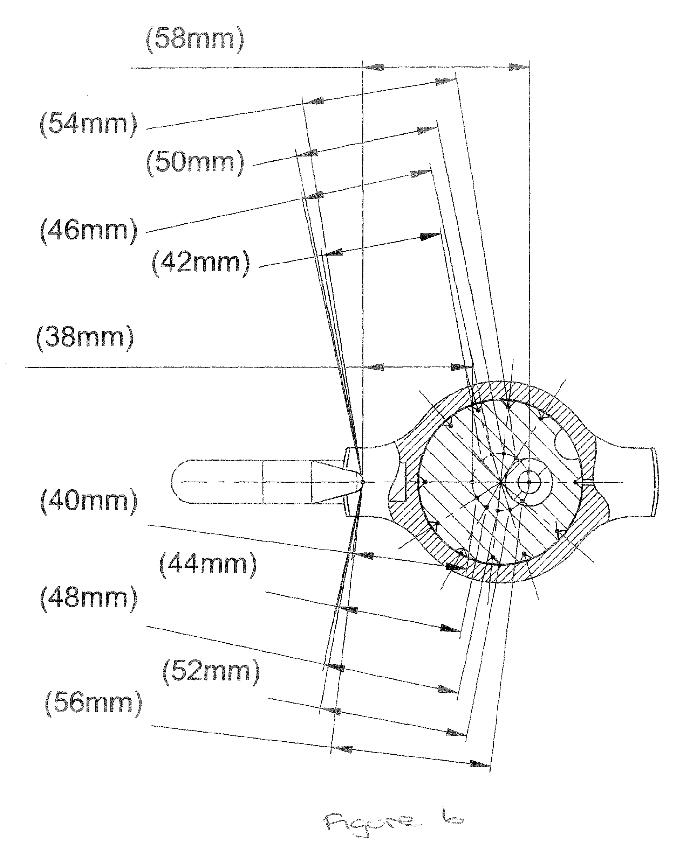

The relationship between the various indexing slots and particular sizes is illustrated graphically in Figure 6.

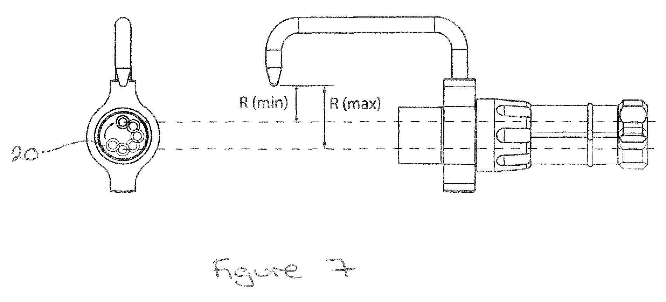

The relationship between the position of the aperture and the tip and the radius is illustrated in Figure 7. The aperture 20 corresponding to the bore of the cannulated rod are movable around an arc. When the aperture is located closest to the point of connection of the collar and the arm represents the smallest radius Rmin and the position furthest away represents the largest radius Rmax.

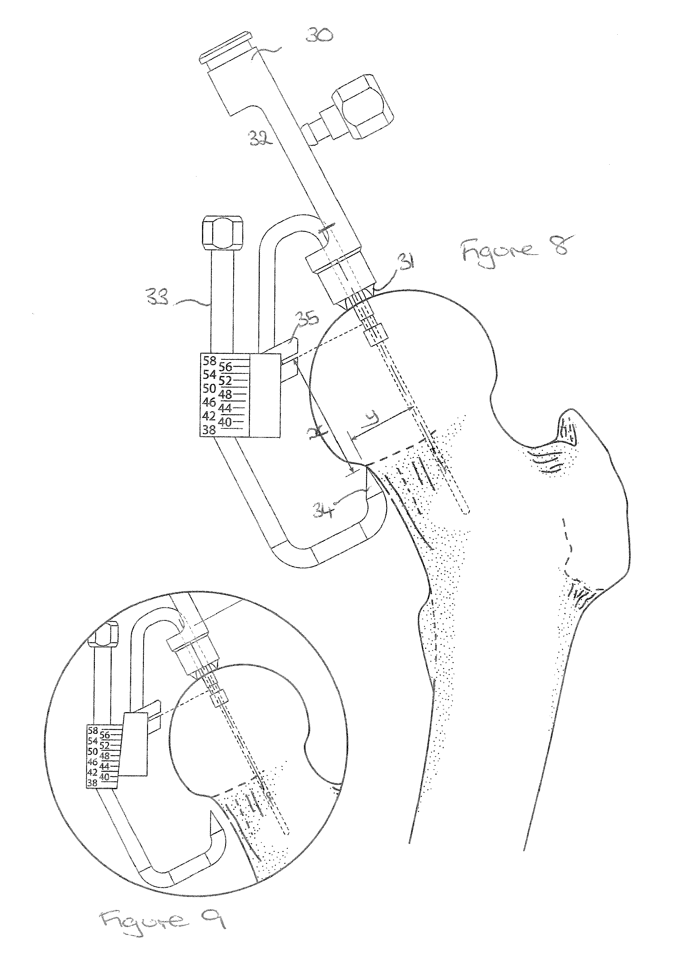

An alternative arrangement is illustrated in Figure 8. Here a cannulated rod 30 is provided which has teeth 31 to assist in holding the rod in position. The arm passes through a window 32 in the wall of the cannulated rod such that it can align with the bore in the head of the femur. The cannulated rod may be the rod from the improved alignment guides that are described in co-pending UK applications 0408792 and 0408793 filed 20th April 2004, 0419494 filed 2nd September 2004 and 0419640 filed 3rd September 2004.

The arm is articulated to enable it to be adjusted as to the radius and the length. The articulation is adjusted by control 33. When the tip 34 is located at the head neck junction, the distance x from the tip 34 to a cutting guide 35. The tip will also be a distance y from the bore for the guide. The tip may then be moved to the position of the cutting as illustrated in Figure 9 and the jig is used to check that the machining will not impinge on the neck.

## Claims

1. A checking jig comprising: a body (2) having an aperture therethrough which in use can be placed over a guide wire located in a well in the head of the femur; an arm (3) extending from the body and shaped to extend around the head of the femur; a tip (4) located on the end of the arm remote from the body, said tip representing the diameter at which a particular size of sleeve cutter will cut; and means for adjusting the position of the tip between different positions representing different cut diameters.

2. A checking jig according to Claim 1 wherein the different positions are a selection of preset positions.

3. A checking jig according to Claim 2 wherein the selection of preset positions correspond to the full range of head sizes available.

4. A checking jig according to any one of Claims 1 to 3 wherein the means for adjusting the position of the tip is located on or within the body.

5. A checking jig according to Claim 4 wherein the body is formed from a fixed first member and a movable second member which is connectable to the first member and movable with respect thereto.

6. A checking jig according to Claim 5 wherein the arm is connected to one of the first and second member and the other of the first and second member includes an aperture through which, in use, the guidewire can be placed.

7. A checking jig according to Claim 6 wherein one of the first and second member is a collar (5) in which the second member is a sliding fit.

8. A checking jig according to any one of Claims 5 to 7 wherein the relative movement is rotational.

9. A checking jig according to any one of Claims 1 to 8 wherein the jig comprises a body which comprises a collar means having an arm extending therefrom which is shaped to extend round the head of a femur and a second member which is a sliding fit in the collar (5).

10. A checking jig according to Claim 9 wherein the second member is a rotatable jog dial (10) which has a skirt that is a sliding fit in the collar.

11. A checking jig according to Claim 9 or 10 wherein means are provided to prevent the second member from being removed from the collar when the jig is in use.

12. A checking jig according to Claim 9 or 10 wherein means to enable the jog dial to move between preset locations is a spring ball grub screw mechanism.

13. A checking jig according to any one of Claims 1 to 12 wherein the body includes an elongated cannulated rod extending upwardly from the body such that the bore of the cannulated rod cooperates with the aperture in the body.

14. A checking jig according to Claim 13 wherein the cannulated rod is telescopic.

15. A checking jig according to Claim 14 wherein a central portion of a slidable telescopic cannulated rod is removable.

16. A checking jig according to Claim 1 wherein the body comprises a cannulated rod.

17. A checking jig according to Claim 16 wherein the cannulated rod includes teeth.

18. A checking jig according to Claim 16 or 17 wherein the arm is connected to the cannulated rod by means of a sleeve around the rod.

19. A checking jig according to Claim 16 or 17 wherein the arm fits into the bore of the rod.

20. A checking jig according to any one of Claims 16 to 19 wherein the adjusting means is located on the arm.

21. A checking jig according to any one of Claims 16 to 20 wherein the arm is adjustable as to the radius that the tip will travel when the jig is rotated and as to the length of the arm.
